# EUROPEAN PATENT SPECIFICATION

(11) **EP 2 327 976 B1**
(45) Date of publication and mention of the grant of the patent: **19.06.2019**
(21) Application number: 10190420.9
(22) Date of filing: 09.11.2010
(51) Int. Cl.: B60W 40/06, B60W 40/068, E01C 23/01, G01J 5/00, G01N 25/72, G01C 11/00, G01N 33/38, G01N 33/42, G01S 13/88, G01S 19/42

(54) **Method and apparatus for evaluating the condition of the pavement of a traffic lane**
Verfahren und Vorrichtung zur Beurteilung des Zustands des Belags einer Fahrbahn
Procédé et appareil permettant d'évaluer l'état de la chaussée d'une voie de circulation

(30) Priority: 20.11.2009 FI 20096217
(43) Date of publication of application: 01.06.2011
(73) Proprietor: Roadscanners Holding Oy, 96100 Rovaniemi (FI)
(72) Inventor: Saarenketo, Timo, 96400 Rovaniemi (FI)
(74) Representative: Berggren Oy, Helsinki & Oulu

(56) References cited:
- JP-A- H0 320 654
- JP-A- 2002 257 744
- US-A- 4 899 296
- US-A- 4 910 593
- SAARENKETO T ET AL: "Road evaluation with ground penetrating radar", JOURNAL OF APPLIED GEOPHYSICS, ELSEVIER, AMSTERDAM, NL, vol. 43, no. 2-4, 27 May 1998 (1998-05-27) , pages 119-138, XP002334958, ISSN: 0926-9851, DOI: 10.1016/S0926-9851(99)00052-X

## Description

The invention relates to a method for evaluating the condition of the pavement of a traffic lane. The invention further relates to an apparatus used in the method.

Ruts, which impede movement on the road, are formed with time at the driving lines in the bitumen pavement of roads and tracks. The mechanical wear of the surface of the pavement caused by studded tires is in Finland and the Nordic countries generally regarded as the main cause of the formation of ruts. Studies have however shown that a significant part of the rutting is caused by permanent deformations occurring in the unbound layer on the underside of the pavement, which deformations can be seen on the surface of the pavement as rutting or cracking. These deformations occur especially in the spring as the frozen road melts and in the fall after hard rains, when a great amount of moisture gets under the pavement. The amount of deformations depends on the tensions prevailing in the layer beneath the pavement, which tensions are affected among others by axle loads and the thickness and stiffness of the pavement. If there is a great amount of moisture beneath the pavement, the pavement can be damaged also when heavy traffic moving on top of the pavement causes a hydrostatic pressure on the lower surface of the pavement, which leads to breaking of the bonds between the bitumen and the rock material. Thus the pavement is damaged from below upwards. In addition to what was said above, frost and freezing can cause damage in the pavement.

The condition of the pavement has a significant impact on the amount of formed damages. The more cracks and gaps, which function as flow routes for the water, there are in the pavement, the larger the amount of water getting under the pavement is. A reliable evaluation of micro and macro cracks in the pavement is of first-rate importance for evaluating the correct time for repairing the pavement.

The most common method for evaluating the condition of the pavement is a visual examination of the condition of the pavement performed by a professional, which examination is performed directly in the field or based on a video taken of the road. In the visual examination the amount and type of cracks in the pavement is determined, and an evaluation of the water-tightness and repair need is done based on these. In visual examination, the reliability of the result of the evaluation varies a lot according to the professional skill of the person doing it.

A ground-penetrating radar can be used in evaluating the condition of the pavement, with which ground-penetrating radar the thickness of the pavement is measured. The thickness of the pavement does not however always correlate directly with the amount of micro and macro cracks in the pavement. Measurements performed with ground-penetrating radars cannot therefore alone generate enough information about the condition of the pavement in order to determine a repair time.

Document US 4910593 A discloses a system for detecting subterranean geological anomalies. Document US 4899296 A discloses a pavement distress survey system.

The object of the invention is to provide a method and an apparatus for evaluating the condition of the pavement of a traffic lane, with which disadvantages and flaws relating to the prior art can be significantly reduced.

The objects of the invention are obtained with a method and an apparatus, which are characterized in what is presented in the independent claims. Some advantageous embodiments of the invention are presented in the dependent claims.

The method according to the invention is meant for evaluating the condition of the pavement of a traffic lane. A traffic lane in this presentation generally means all areas meant for vehicular traffic, such as yards, parking areas and especially roads, tracks and streets, which are paved with a wear-resistant heavy-duty pavement, such as asphalt, light asphalt or tarmac. Though the invention is especially well suited for evaluating the condition of pavements containing bitumen as a binder, it can be applied also for evaluating the condition of other traffic lane pavements, such as concrete pavements.

When there are cracks in the pavement of a traffic lane, the water on top of the pavement can flow through the cracks beneath the pavement onto the bearing course. The bearing course can absorb water with the aid of its suction pressure properties also from lower courses and from the verge of the road. Especially in spring when the surface of the roads melts, the bearing course, typically a layer of gravel or crushed stone, is still frozen, wherefore water passing under the pavement is not absorbed through the bearing course into lower soil layers. Water passing under the pavement thus remains immediately underneath the pavement. The situation is similar in the fall, when a lot of water rains onto the surface of traffic lanes of the road. Water passing beneath the pavement through cracks in the pavement does thus not have time to absorb completely into the lower courses and a part of the water remains in the surface part of the bearing course. In the summer the courses of traffic lanes are typically so dry that water passing through the pavement is usually absorbed quickly into the lower courses, whereby water does not accumulate underneath the pavement to a significant degree.

The method according to the invention is based on the observation that water beneath the pavement, on top of the bearing course, is colder than the sun-warmed pavement above it, i.e. the temperature of the water beneath the pavement is lower than the temperature of the pavement itself. The water beneath the pavement lowers the temperature of the pavement, when it is pressed into micro and macro gaps in the pavement due to the force of the traffic load. By measuring it has been discovered that if there is water in the top part of the bearing course beneath the cracked pavement, the pavement can be 0.5-2.0 °C lower than in a corresponding pavement, which does not contain micro or macro cracks or the course beneath which is essentially dry. The above-mentioned temperature difference of 0.5-2.0 °C is so large that it can easily be observed with temperature measuring devices in general use, such as a precision thermal camera, a thermal scanner or temperature sensors. In order to eliminate the heating effect of the sunshine on the pavement, the measurements should be performed in the evening or at night or on a cloudy day.

In the method according to the invention, an examination span, the condition of the pavement of which is evaluated, is first chosen. The length of the examination span can be freely chosen. The examination span can at its shortest be a few meters and at its longest tens of meters. It is natural to divide a long traffic lane into several successive examination spans. Thereafter a temperature T1 of a heavily loaded part of the pavement of the selected examination span and a temperature T2 of a lightly loaded part of the pavement of the same examination span are determined. Alternatively an emissivity ε₁ of the heavily loaded part of the pavement of the selected examination span and an emissivity ε₂ of the lightly loaded part of the pavement of the same examination span can be determined. A difference ΔT or Δε is calculated from the determined temperatures or emissivities, which difference is compared to a reference value. If the difference ΔT or Δε is significant, i.e. it is larger than the used reference value, this is a sign that there are micro and/or macro cracks in the pavement and a significant amount of water can pass through the pavement. Steps to repair the pavement should thus be taken at the examination span in question. If, on the other hand, the difference ΔT or Δε is not significant, i.e. it is smaller than the used reference value, this means that the condition of the pavement is sufficiently good. The pavement in the examination span is thus not in immediate need of restoration or repair.

In an advantageous embodiment of the method according to the invention the temperature T1 of the heavily loaded part of the pavement is determined by measuring the temperature *tᵣ* of the pavement in one measuring point within the examination span and the temperature T2 of the lightly loaded part of the pavement is determined by measuring the temperature *tₖ* of the pavement in one measuring point within the examination span. The measurement results thus give temperature information directly describing the entire examination span. Such a measuring manner can be used in cases where the selected examination span is very short, and in cases where long sections of traffic lanes are examined using random testing.

In another advantageous embodiment of the method according to the invention the temperature *tᵣ* of the heavily loaded part of the pavement is measured at more than one measuring point within the examination span and in the same way the temperature *tₖ* of the lightly loaded part of the pavement is also measured at more than one measuring point within the examination span. The temperatures T1 and T2 describing the examination span are determined from these measured temperatures, for example as an arithmetic average or median value of the measurement results. The highest and/or lowest values can also be removed from the measurement results in order to eliminate possible erroneous results and the temperatures T1 and T2 can be determined as the average of the remaining measurement results.

In a third advantageous embodiment of the method according to the invention the temperature *tᵣ* of the heavily loaded part of the pavement is measured at the wheel ruts of the traffic lane and the temperature *tₖ* of the lightly loaded part of the pavement is measured in the area between the wheel ruts or in the area between a wheel rut and the verge of the pavement. The temperatures of the pavement are advantageously measured from a moving vehicle travelling on the traffic lane.

It is clear that the coatings of traffic lanes are damaged at first in the parts that are heavily loaded. In roads and streets these heavily loaded parts are the wheel ruts of the vehicles, i.e. the parts of the pavement of the road or street, where the load of the wheels of the vehicles is directed. Wheel ruts here thus mean a certain area of the cross-section of the traffic lane, which is mainly loaded by the wheels of vehicles. Physical ruts are often formed at the wheel ruts, due to wear in the surface of the pavement and deformations of the bearing structure. The wheel rut does however not necessarily need to be a concave part of the cross-section of the traffic lane, but its surface can also be substantially even.

There are typically two wheel ruts per driving lane, whereby a substantially more lightly loaded area is left between the wheel ruts. Similarly a substantially more lightly loaded area outside the wheel rut is left between the outermost wheel rut and the verge of the pavement, which is toward the shoulder of the road.

In a fourth advantageous embodiment of the method according to the invention the location data of the measuring points of the temperatures tᵣ and tₖ or the examination span is also determined. The thickness of the pavement is advantageously also determined in the method.

The apparatus according to the invention for evaluating the condition of the pavement of a traffic lane comprises temperature measuring means for measuring the temperature t*ᵣ* or the emissivity ε*ᵣ* of a heavily loaded part of the pavement and the temperature t*ₖ* or the emissivity ε*ₖ* of a lightly loaded part of the pavement, locating means for determining the location data of the measuring points of the temperatures tᵣ and tₖ or the emissivities εᵣ and εₖ and/or the examination span of the traffic lane, and storage and analysis means for storing and analyzing the measured temperatures tᵣ, tₖ, the emissivities εᵣ, εₖ, and the location data. Said storage and analysis means are configured to determine a temperature T1 or emissivity ε₁ of the heavily loaded part of the pavement and a temperature T2 or emissivity ε₂ of the lightly loaded part of the pavement, calculate the difference between said temperatures or emissivities (ΔT or Δε), and compare the difference to a reference value for forming an evaluation of the condition of the pavement. Said temperature measuring means can comprise a thermal camera, a thermal scanner or several temperature measuring sensors, which can be manoeuvred above the pavement. Said locating means advantageously comprise a GPS locator.

An advantageous embodiment of the apparatus according to the invention further comprises a ground-penetrating radar for measuring the thickness of the pavement. The apparatus can further include a video camera for filming the surface of the pavement. The apparatus is advantageously placed in a vehicle, which can be used to move along the traffic lane to be examined.

It is an advantage of the invention that damages in the pavement can by means of it be discovered at a very early stage, whereby the formation of further damages can often be prevented with simple and inexpensive maintenance operations, such as by improving drying or a new water-tight coating.

It is a further advantage of the invention that a more reliable evaluation of the condition of the pavement can be obtained by means of it than with presently used methods.

It is an advantage of an embodiment of the invention that the evaluation of the condition of the pavement can be performed with a moving vehicle, which makes possible the evaluation of the condition of long stretches of road in a quick and inexpensive manner.

In the following, the invention will be described in detail. In the description, reference is made to the appended drawings, in which
Figure 1 shows as an example a method according to the invention as a simple flow chart,
Figure 2a shows as an example an apparatus used in the method according to the invention seen from the side and
Figure 2b shows the apparatus of Figure 2a seen from the front.

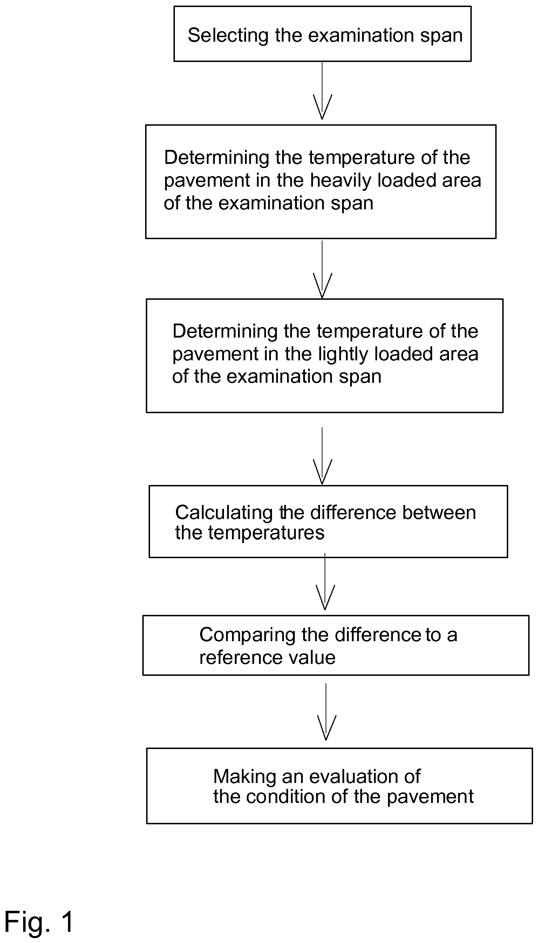

Figure 1 shows as an example the steps of a method according to the invention with the aid of a simple flow chart. In the method according to the invention the condition of the pavement of a traffic lane is always evaluated over a certain examination span by measuring the temperature of the pavement at different points of the cross-section of the examination span. In the method, the examination span of the traffic lane to be examined is first selected. Examination span means the stretch between two points on a traffic lane, such as a road, a street or a track. The length of the examination span can be freely chosen.

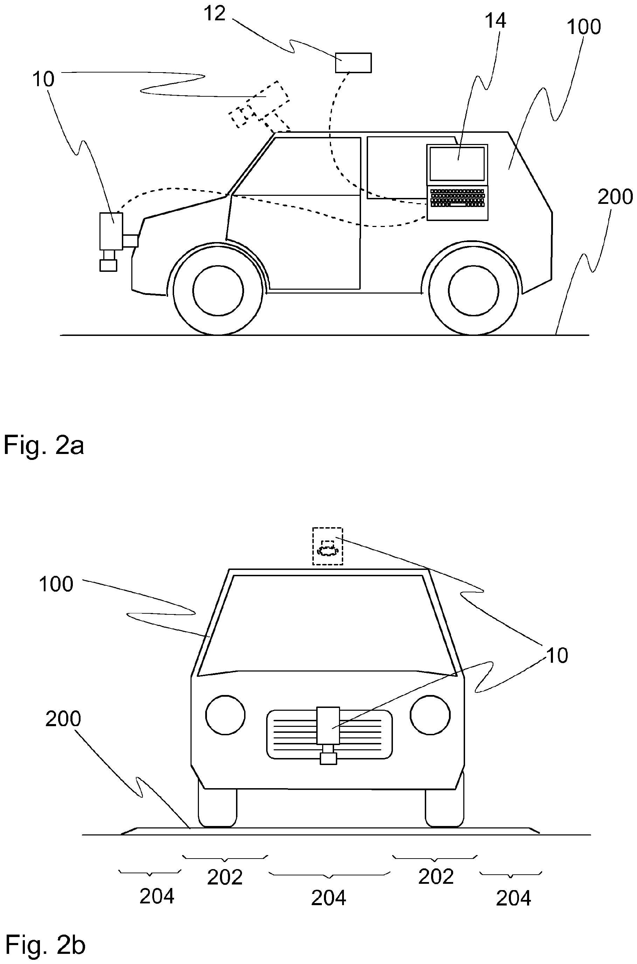

Thereafter the temperature t*ᵣ* of the pavement is measured in at least one point in the heavily loaded area 202 of the examination span (Figure 2b). Typically such a heavily loaded part of the pavement of a traffic lane is the wheel rut of a road, track or street. Thereafter the temperature t*ₖ* of the pavement is measured in at least one point in the lightly loaded area 204 of the examination span (Figure 2b). Typically such lightly loaded areas are the areas between the wheel ruts and the areas between the wheel ruts and the verges of the paved area. The measuring order of the temperatures has no importance, i.e. the temperatures in the lightly loaded area of the examination span can just as easily be measured first and the temperatures of the heavily loaded area thereafter. After the measurements, the temperatures T1, T2 of the heavily and lightly loaded areas, which describe the examination span, are determined for example as an arithmetic average of the individual measurement results and their difference ΔT is calculated. This difference is compared to the obtained reference value. If ΔT is higher than the reference value, the condition of the pavement of the examination span is poor, i.e. the pavement is in need of repair. If the difference is lower than the reference value, the pavement is in no immediate need of repair.

The reference value used in the method decides what kind of evaluation the condition of the pavement receives. It has been discovered in studies that when the temperature difference ΔT is higher than 2.0 °C, the waterproofing ability of the pavement is already clearly reduced, i.e. the condition of the pavement is poor. It has been discovered that the thickness of the pavement layer also affects the temperature difference ΔT. The thickness of the pavement on traffic lanes is typically about 50-150 mm, but it can vary in the range of 30-300 mm. In thicker pavement layers the temperature difference is smaller than in thinner pavement layers. In thick pavements a temperature difference of 0.5 °C can already be a sign that the pavement has micro or macro cracks and that the waterproofing ability of the pavement is poor. Therefore different values can be used as reference values depending on how thick the pavement is. The reference value used in the method can thus be 0.5, 1.0, 1.5 or 2.0 °C. The thickness of the pavement can be measured for example with the aid of a ground-penetrating radar simultaneously with the temperature measurements.

Figure 2a shows as an example an apparatus to be used in the method according to the invention seen from the side, and Figure 2b shows it from the front. The apparatus according to the invention is placed in a vehicle 100, such as an ordinary passenger car or van. The necessary measurements are performed with the apparatus during driving, i.e. as the vehicle moves along the traffic lane to be examined.

The apparatus comprises temperature measuring means 10, which can be used to measure the temperature of the pavement 200 of the traffic lane at different points on the cross-section of the examination span. Such a measuring means can for example be a thermal camera, preferably a so-called precision thermal camera or a thermal scanner. The precision thermal camera and the thermal scanner register the intensity of the thermal radiation emitted from the pavement, based on which intensity they calculate the temperature of the pavement. The structure and operating principle of thermal cameras and thermal scanners are generally known as such, hence they are not described in more detail in this context. The measuring area of the thermal camera or thermal scanner is usually so wide that it covers two parallel wheel ruts of the driving lane, and the area remaining between them. The temperature t*ᵣ* of the heavily loaded area 202 and the temperature t*ₖ* of the lightly loaded area 204 can thus be measured simultaneously with one device (Figure 2b). If the measuring area of the thermal camera or thermal scanner is not sufficient, the apparatus according to the invention can be equipped with more than one thermal scanner or thermal camera, for example with two thermal scanners or thermal cameras placed by the wheels of the vehicle used in the measuring, in order to obtain a sufficiently wide measuring area extending outside the area limited between the wheel ruts.

Corresponding evaluations of the condition of the pavement can be made also based on the emissivity of the pavement. The emissivity of a surface is a variable, which states the ratio between the radiation emitted by the surface and the radiation emitted by a black object. On real surfaces the emissivity of the surface is not constant, but it varies as a function of the temperature, radiation wavelength and radiation direction of the surface. The emissivity of the pavement can thus also be calculated from the measurement data of the thermal camera or thermal scanner, when the measuring angle between the measuring direction of the thermal camera and the surface of the pavement and the wavelength of the radiation is known. The emissivity can, just as the temperature, be given a reference value, to which obtained measurement results are compared. In an alternative embodiment of the method according to the invention, instead of determining the temperatures, the emissivity ε*ᵣ* of the heavily loaded area of the pavement and the emissivity ε*ₖ* of the lightly loaded area of the pavement are determined over the examination span, and the difference between the emissivities Δε is calculated. This calculated difference is compared to a reference value for the emissivity and an evaluation of the condition of the pavement is made based on the comparison. The determination of emissivity is performed from the measurement data measured with the thermal camera or thermal scanner. In this embodiment of the invention the thermal camera can be attached in a slanting position in relation to the surface of the pavement. This makes possible the attaching of the thermal camera for example on the roof of the vehicle and the directing of the measuring area slantingly downwards and forwards, whereby the width of the measuring area grows.

The temperature measuring means can also comprise several parallel temperature measuring sensors at a distance from each other. The measuring sensors can for example be infrared measuring sensors, which measure infrared radiation emitted by the pavement. There can be a laser device in connection with the measuring sensors, with which laser device a laser beam is produced onto the surface of the pavement, which laser beam facilitates the alignment of the measuring point. The measuring sensors are advantageously placed on a horizontal beam attached to the vehicle, which in a measuring situation moves above the surface of the traffic lane to be examined, when the vehicle is driven along the traffic lane.

The apparatus includes a locating means 12 for determining the location of the measuring point. In the apparatus shown in Figures 2a and 2b the locating means is a GPS locator, which continuously states the location of the measuring apparatus with the aid of coordinates. The location data of the measuring point is always added to each measured temperature value. This location data can be the precise location of an individual measuring point or it can be an "average" location data describing the examination span. The locating means used for determining the location of a measuring point can also be for example a distance measuring means, such as the trip mileage indicator, so-called trip meter, of a vehicle, with which the distance from some starting point, such as a crossroads, is measured. So-called optical encoders used in ground-penetrating radars can also be used for the locating.

The apparatus further includes a storage and analysis means 14 for the measured data, wherein the measured temperature data and thereto belonging location data are stored. In the apparatus shown in Figures 2a and 2b the storage and analysis means is a laptop, which has a memory for storing the data and a processor and required computer software for analyzing the data. The computer naturally further also has parts generally belonging to computers, which are important for its operation and use, such as a monitor, a keyboard, an operating system etc. The structure of computers is known as such, which is not described in further detail in this context. The computer is connected with a wireless or wired data transfer connection to the temperature measuring means 10 and the locating means 12, whereby the measured temperatures t*ᵣ*, t*ₖ* are automatically stored in the memory of the computer.

The apparatus can further comprise a ground-penetrating radar, with which the thickness of the pavement is measured simultaneously with the temperature measurements. The measuring of the thickness of the pavement gives additional information about the condition of the pavement and thus affects the evaluation of the repair need and repair timetable. The use of a ground-penetrating radar in measuring the thickness of the pavement is known as such, hence it is not described in further detail in this context.

The apparatus can further include a video camera, with which the surface of the pavement to be examined is filmed. The image of the video camera makes possible a visual examination of the surface of the pavement. Visual examination can be used alongside the method based on measurements described above, especially in unclear borderline cases that are up for interpretation.

Some advantageous embodiments of the method and apparatus according to the invention have been described above. The invention is not limited to the solutions described above, but the inventive idea can be applied in numerous ways within the scope of the invention. The scope of the invention is defined by the appended claims.

## Claims

1. A method for evaluating the condition of the pavement (200) of a traffic lane, **characterized in that** the method comprises:
- selecting an examination span of the traffic lane,
- determining a temperature T1 or emissivity ε₁ of a heavily loaded part of the pavement (200) of the examination span,
- determining a temperature T2 or emissivity ε₂ of a lightly loaded part of the pavement of the examination span,
- calculating a difference ΔT between the temperatures T1 and T2 or a difference Δε between the emissivities ε₁ and ε₂, and
- forming an evaluation of the condition of the pavement (200) of the examination span by comparing the difference ΔT or Δε to a reference value.

2. The method according to claim 1, **characterized in**
- determining the temperature T1 of the heavily loaded part of the pavement (200) by measuring the temperature *tᵣ* of the pavement (200) in one measuring point in the examination span, and
- determining the temperature T2 of the lightly loaded part of the pavement (200) by measuring the temperature *tₖ* of the pavement (200) in one measuring point in the examination span.

3. The method according to claim 1, **characterized in**
- measuring the temperature *tᵣ* of the heavily loaded part of the pavement (200) in more than one measuring point over the examination span and determining the temperature T1 from the measured temperatures tᵣ, and
- measuring the temperature *tₖ* of the lightly loaded part of the pavement (200) in more than one measuring point over the examination span and determining the temperature T2 from the measured temperatures *tₖ.*

4. The method according to claim 2 or 3, **characterized in** measuring the temperature *tᵣ* of the heavily loaded part of the pavement (200) at the wheel ruts of the traffic lane and measuring the temperature tₖ of the lightly loaded part of the pavement (200) in the area between the wheel ruts or in the area between a wheel rut and the verge of the pavement (200).

5. The method according to any one of the claims 2-4, **characterized in** measuring the temperatures tᵣ and tₖ of the pavement (200) from a moving vehicle (100) travelling on the traffic lane.

6. The method according to any one of the claims 2-5, **characterized in** further determining the location data of the measuring points of the temperatures tᵣ and tₖ or the examination span.

7. The method according to any one of the claims 1-6, **characterized in** further determining the thickness of the pavement (200).

8. The method according to claim 7 when claim 7 refers back to claim 6 **characterized in** storing the measured temperatures tᵣ and tₖ, the location data of the measuring points or the examination span, and the thickness data of the pavement (200) in a storage and analysis means (14).

9. An apparatus for evaluating the condition of the pavement (200) of a traffic lane, which apparatus comprises:
- temperature measuring means (10) for measuring the temperature *tᵣ* or the emissivity ε*ᵣ* of a heavily loaded part of the pavement (200) and the temperature t*ₖ* or the emissivity ε*ₖ* of a lightly loaded part of the pavement (200),
- locating means (12) for determining the location data of the measuring points of the temperatures tᵣ and tₖ or the emissivities εᵣ and εₖ and/or the examination span of the traffic lane, and
- storage and analysis means (14) for storing and analyzing the measured temperatures *tᵣ*, t*ₖ*, the emissivities ε*ᵣ*, εₖ, and the location data,
**characterized in that**
said storage and analysis means (14) are configured to:
- determine a temperature T1 or emissivity ε₁ of the heavily loaded part of the pavement (200) and a temperature T2 or emissivity ε₂ of the lightly loaded part of the pavement (200),
- calculate the difference between said temperatures or emissivities (ΔT or Δε), and
- compare the difference to a reference value for forming an evaluation of the condition of the pavement (200).

10. The apparatus according to claim 9, **characterized in that** said temperature measuring means (10) for measuring the temperatures tᵣ and tₖ or the emissivities ε*ᵣ*, ε*ₖ* comprise a thermal camera or a thermal scanner.

11. The apparatus according to claim 9, **characterized in that** said temperature measuring means (10) comprise several temperature measuring sensors, which can be manoeuvred above the pavement (200).

12. The apparatus according to any one of the claims 9-11, **characterized in that** said locating means (12) comprise a GPS locator.

13. The apparatus according to any one of the claims 9-12, **characterized in that** it further comprises a ground-penetrating radar for measuring the thickness of the pavement (200).

14. The apparatus according to any one of the claims 9-13, **characterized in that** it further comprises a video camera for filming the surface of the pavement (200).

15. The apparatus according to any one of the claims 9-14, **characterized in that** it is placed in a vehicle (100).

## Patentansprüche

1. Verfahren zur Beurteilung des Zustands des Belags (200) einer Fahrspur, **dadurch gekennzeichnet, dass** das Verfahren Folgendes umfasst:
- Auswählen einer Überprüfungsspanne der Fahrspur,
- Feststellen einer Temperatur T1 oder eines Emissionsgrades ε₁ eines stark belasteten Teils des Belags (200) der Überprüfungsspanne,
- Feststellen einer Temperatur T2 oder eines Emissionsgrades ε₂ eines leicht belasteten Teils des Belags der Überprüfungsspanne,
- Berechnen einer Differenz ΔT zwischen den Temperaturen T1 und T2 oder einer Differenz Δε zwischen den Emissionsgraden ε₁ und ε₂, und
- Ausbilden einer Bewertung des Zustands des Belags (200) der Überprüfungsspanne durch Vergleichen der Differenz ΔT oder Δε mit einem Referenzwert.

2. Verfahren nach Anspruch 1, **gekennzeichnet durch**
- Feststellen der Temperatur T1 des stark belasteten Teils des Belags (200) durch Messen der Temperatur tᵣ des Belags (200) in einem Messpunkt in der Überprüfungsspanne, und
- Feststellen der Temperatur T2 des leicht belasteten Teils des Belags (200) durch Messen der Temperatur tₖ des Belags (200) in einem Messpunkt in der Überprüfungs spanne.

3. Verfahren nach Anspruch 1, **gekennzeichnet durch**
- Messen der Temperatur tᵣ des stark belasteten Teils des Belags (200) in mehr als einem Messpunkt über die Überprüfungsspanne und Feststellen der Temperatur T1 auf der Grundlage der gemessenen Temperaturen tᵣ*,* und
- Messen der Temperatur tₖ des leicht belasteten Teils des Belags (200) in mehr als einem Messpunkt über die Überprüfungsspanne und Feststellen der Temperatur T2 auf der Grundlage der gemessenen Temperaturen tₖ.

4. Verfahren nach Anspruch 2 oder 3, **gekennzeichnet durch** ein Messen der Temperatur tᵣ des stark belasteten Teils des Belags (200) an den Radrinnen der Fahrspur und Messen der Temperatur tₖ des leicht belasteten Teils des Belags (200) in dem Bereich zwischen den Radrinnen oder in dem Bereich zwischen einer Radrinne und dem Rand des Belags (200).

5. Verfahren nach einem der Ansprüche 2-4, **gekennzeichnet durch** Messen der Temperaturen tᵣ und tₖ des Belags (200) von einem sich bewegenden Fahrzeug (100), das auf der Fahrspur fährt.

6. Verfahren nach einem der Ansprüche 2-5, ferner **gekennzeichnet durch** Bestimmen der Positionsdaten der Messpunkte der Temperaturen tᵣ und tₖ oder der Überprüfungs spanne.

7. Verfahren nach einem der Ansprüche 1-6, ferner **gekennzeichnet durch** Feststellen der Dicke des Belags (200) .

8. Verfahren nach Anspruch 7, wenn Anspruch 7 zurück auf Anspruch 6 verweist, **gekennzeichnet durch** Speichern der gemessenen Temperaturen tᵣ und tₖ, der Positionsdaten der Messpunkte oder der Überprüfungsspanne und der Dickedaten des Belags (200) in einem Speicher- und Analysemittel (14).

9. Vorrichtung zum Bewerten des Zustands des Belags (200) einer Fahrspur, wobei die Vorrichtung Folgendes umfasst:
- Temperaturmessmittel (10) zum Messen der Temperatur tᵣ oder des Emissionsgrades εᵣ eines stark belasteten Teils des Belags (200) und der Temperatur tₖ oder des Emissionsgrades εₖ eines leicht belasteten Teils des Belags (200),
- Lokalisierungsmittel (12) zum Feststellen der Positionsdaten der Messpunkte der Temperaturen tᵣ und tₖ oder der Emissionsgrade εᵣ und εₖ und/oder der Überprüfungsspanne der Fahrspur, und
- Speicher- und Analysemittel (14) zum Speichern und Analysieren der gemessenen Temperaturen tᵣ und tₖ, der Emissionsgrade εᵣ und εₖ und der Positionsdaten,
**dadurch gekennzeichnet, dass**
die Speicher- und Analysemittel (14) dazu ausgelegt sind:
- eine Temperatur T1 oder einen Emissionsgrad ε₁ des stark belasteten Teils des Belags (200) und eine Temperatur T2 oder einen Emissionsgrad ε₂ des leicht belasteten Teils des Belags (200) festzustellen,
- die Differenz zwischen den Temperaturen oder Emissionsgraden (ΔT oder Δε) zu berechnen, und
- die Differenz mit einem Referenzwert zu vergleichen, um eine Bewertung des Zustands des Belags (200) auszubilden.

10. Vorrichtung nach Anspruch 9, **dadurch gekennzeichnet, dass** das Temperaturmessmittel (10) zum Messen der Temperaturen tᵣ und tₖ oder der Emissionsgrade εᵣ, εₖ eine thermische Kamera oder einen thermischen Scanner umfasst.

11. Vorrichtung nach Anspruch 9, **dadurch gekennzeichnet, dass** das Temperaturmessmittel (10) mehrere Temperaturmesssensoren umfasst, die über dem Belag (200) manövriert werden können.

12. Vorrichtung nach einem der Ansprüche 9-11, **dadurch gekennzeichnet, dass** das Lokalisierungsmittel (12) einen GPS-Positionsgeber umfasst.

13. Vorrichtung nach einem der Ansprüche 9-12, **dadurch gekennzeichnet, dass** sie ferner ein den Boden penetrierendes Radar zum Messen der Dicke des Belags (200) umfasst.

14. Vorrichtung nach einem der Ansprüche 9-13, **dadurch gekennzeichnet, dass** sie ferner eine Videokamera zum Filmen der Oberfläche des Belags (200) umfasst.

15. Vorrichtung nach einem der Ansprüche 9-14, **dadurch gekennzeichnet, dass** sie in einem Fahrzeug (100) platziert ist.

## Revendications

1. Procédé permettant d'évaluer l'état de la chaussée (200) d'une voie de circulation,
**caractérisé en ce que** le procédé comprend les étapes consistant à :
sélectionner une portée examinée de la voie de circulation,
déterminer une température T1 ou émissivité ε₁ d'une partie fortement chargée de la chaussée (200) de la portée examinée,
déterminer une température T2 ou émissivité ε₂ d'une partie légèrement chargée de la chaussée de la portée examinée,
calculer une différence ΔT entre les températures T1 et T2 ou une différence Δε entre les émissivités ε₁ et ε₂, et
former une évaluation de l'état de la chaussée (200) de la portée examinée en comparant la différence ΔT ou Δε à une valeur de référence.

2. Procédé selon la revendication 1, **caractérisé par** les étapes consistant à
déterminer la température T1 de la partie fortement chargée de la chaussée (200) en mesurant la température *tᵣ* de la chaussée (200) en un point de mesure dans la portée examinée, et
déterminer la température T2 de la partie légèrement chargée de la chaussée (200) en mesurant la température *tₖ* de la chaussée (200) en un point de mesure dans la portée examinée.

3. Procédé selon la revendication 1, **caractérisé par** les étapes consistant à
mesurer la température *tᵣ* de la partie fortement chargée de la chaussée (200) en plus d'un point de mesure sur la portée examinée et déterminer la température T1 à partir des températures mesurées *tᵣ*, et
mesurer la température *tₖ* de la partie légèrement chargée de la chaussée (200) en plus d'un point de mesure sur la portée examinée et déterminer la température T2 à partir des températures mesurées *tₖ.*

4. Procédé selon la revendication 2 ou 3, **caractérisé en ce qu'**il comprend les étapes consistant à mesurer la température *tᵣ* de la partie fortement chargée de la chaussée (200) au niveau des ornières de la voie de circulation et à mesurer la température *tₖ* de la partie légèrement chargée de la chaussée (200) dans la zone entre les ornières ou dans la zone entre une ornière et le bord de la chaussée (200).

5. Procédé selon l'une quelconque des revendications 2 à 4, **caractérisé en ce qu'**il comprend l'étape consistant à mesurer les températures *tᵣ* et *tₖ* de la chaussée (200) à partir d'un véhicule en mouvement (100) circulant sur la voie de circulation.

6. Procédé selon l'une quelconque des revendications 2 à 5, **caractérisé en ce qu'**il comprend l'étape consistant à déterminer en outre les données de localisation des points de mesure des températures *tᵣ* et *tₖ* ou de la portée examinée.

7. Procédé selon l'une quelconque des revendications 1 à 6, **caractérisé en ce qu'**il comprend en outre l'étape consistant à déterminer l'épaisseur de la chaussée (200).

8. Procédé selon la revendication 7 lorsque la revendication 7 renvoie à la revendication 6, **caractérisé en ce qu'**il comprend l'étape consistant à mémoriser les températures mesurées *tᵣ* et *tₖ*, les données de localisation des points de mesure ou de la portée examinée, et les données d'épaisseur de la chaussée (200) dans un moyen de mémorisation et d'analyse (14).

9. Appareil permettant d'évaluer l'état de la chaussée (200) d'une voie de circulation, ledit appareil comprenant :
un moyen de mesure de température (10) pour mesurer la température *tᵣ* ou l'émissivité εᵣ d'une partie fortement chargée de la chaussée (200) et la température *tₖ* ou l'émissivité εₖ d'une partie légèrement chargée de la chaussée (200),
un moyen de localisation (12) pour déterminer les données de localisation des points de mesure des températures *tᵣ* et *tₖ* ou des émissivités εᵣ et εₖ et/ou la portée examinée de la voie de circulation, et
un moyen de mémorisation et d'analyse (14) pour mémoriser et analyser les températures mesurées *tᵣ*, *tₖ*, les émissivités εᵣ, εₖ, et les données de localisation,
**caractérisée en ce que**
ledit moyen de mémorisation et d'analyse (14) est conçu pour :
déterminer une température T1 ou émissivité ε₁ de la partie fortement chargée de la chaussée (200) et une température T2 ou émissivité ε₂ de la partie légèrement chargée de la chaussée (200),
calculer la différence entre lesdites températures ou émissivités (ΔT ou Δε), et
comparer la différence à une valeur de référence pour évaluer l'état de la chaussée (200).

10. Appareil selon la revendication 9, **caractérisé en ce que** ledit moyen de mesure de température (10) pour mesurer les températures *tᵣ* et *tₖ* ou les émissivités εᵣ, εₖ, comprend une caméra thermique ou un scanner thermique.

11. Appareil selon la revendication 9, **caractérisé en ce que** ledit moyen de mesure de température (10) comprend plusieurs capteurs de mesure de température, qui peuvent être manoeuvrés au-dessus de la chaussée (200).

12. Appareil selon l'une quelconque des revendications 9 à 11, **caractérisé en ce que** ledit moyen de localisation (12) comprend un localisateur GPS.

13. Appareil selon l'une quelconque des revendications 9 à 12, **caractérisé en ce qu'**il comprend en outre un radar pénétrant dans le sol pour mesurer l'épaisseur de la chaussée (200).

14. Appareil selon l'une quelconque des revendications 9 à 13, **caractérisé en ce qu'**il comprend en outre une caméra vidéo pour filmer la surface de la chaussée (200).

15. Appareil selon l'une quelconque des revendications 9 à 14, **caractérisé en ce qu'**il est placé dans un véhicule (100) .
